# EUROPEAN PATENT SPECIFICATION

(11) **EP 3 638 328 B1**
(45) Date of publication and mention of the grant of the patent: **25.10.2023**
(21) Application number: 18735713.2
(22) Date of filing: 08.06.2018
(51) Int. Cl.: A61L 27/18, A61L 27/34, A61L 27/56, A61L 27/58

(54) **HIERARCHICAL MULTISCALE ELECTROSPUN SCAFFOLD FOR THE REGENERATION AND/OR REPLACEMENT OF THE TENDINOUS/LIGAMENTOUS TISSUE AND A METHOD FOR ITS PRODUCTION**
HIERARCHISCHES MULTISKALIGES ELEKTROGESPONNENES GERÜST FÜR REGENERIERUNG UND/ODER ERSATZ DES SEHNEN-/BÄNDERGEWEBES UND VERFAHREN ZU SEINER HERSTELLUNG
ÉCHAFAUDAGE ÉLECTROFILÉ À STRUCTURE MULTI-ÉCHELLES HIÉRARCHIQUE POUR LA RÉGÉNÉRATION ET/OU LE REMPLACEMENT DU TISSU TENDINEUX/LIGAMENTEUX ET SON PROCÉDÉ DE PRODUCTION

(30) Priority: 12.06.2017 IT 201700064613
(43) Date of publication of application: 22.04.2020
(73) Proprietor: Alma Mater Studiorum - Università di Bologna, 40126 Bologna (IT)
(72) Inventor: SENSINI, Alberto, 47521 Cesena (IT); CRISTOFOLINI, Luca, 40125 Bologna (IT); GUALANDI, Chiara, 40062 Molinella BO (IT); FOCARETE, Maria Letizia, 40131 Bologna (IT); BELCARI, Juri, 41013 Castelfranco Emilia MO (IT); ZUCCHELLI, Andrea, 40014 Crevalcore BO (IT)
(74) Representative: Di Giovine, Paolo
(86) International application number: PCT/IB2018/054153
(87) International publication number: WO 2018/229615

(56) References cited:
- WO-A1-2010/062297
- GB-A- 2 466 073
- ALBERTO SENSINI ET AL: "Biofabrication of bundles of poly(lactic acid)-collagen blends mimicking the fascicles of the human Achille tendon", BIOFABRICATION, vol. 9, no. 1, 8 March 2017 (2017-03-08), page 015025, XP055446261, DOI: 10.1088/1758-5090/aa6204
- BRITTANY L. BANIK ET AL: "Multiscale Poly-([epsilon]-caprolactone) Scaffold Mimicking Non-linearity in Tendon Tissue Mechanics", REGENERATIVE ENGINEERING AND TRANSLATIONAL MEDICINE, vol. 2, no. 1, 25 January 2016 (2016-01-25), pages 1-9, XP055446056, ISSN: 2364-4133, DOI: 10.1007/s40883-016-0008-5

## Description

### Technical field of the invention

The present invention relates to a support (*scaffold*) characterized by a hierarchical and multiscale three-dimensional structure for the tissue regeneration, in particular for the regeneration or replacement of the tendinous and/or ligamentous tissue, as defined in the appended set of claims.

The present invention can also be applied for the regeneration and/or replacement and/or simulation of the muscular and/or nervous tissue.

The present invention further relates to the processes to obtain such support and uses thereof as defined in the appended set of claims.

### State of art

In the field of tissue engineering, the scaffold plays an important role in providing an ideal environment for the adhesion, proliferation and migration of cells. The morphology and the structure of the supports for tissue engineering are fundamental for the shape and definite structure of the tissues and the organs to be reconstructed or replaced. Therefore, there are some specific requirements in structure, in morphology and in other aspects of the physical and chemical properties of the scaffold which make it ideal for the tissue reconstruction and/or replacement.

In particular the *scaffolds* for the reconstruction of the ligament or tendon should be biodegradable, porous, biocompatible, they should have a sufficient resistance and mechanical stiffness, and favour the formation of tissues of ligament or tendon.

Dawei et al. in J. Mater. Chem 8 2015, 3, 8823 describe a support *(scaffold)* for the regeneration of peripheral nervous lesions prepared by electrospinning nanofibers di poly(L-lactide-co-caprolactone) (P(LLA-CL)) and polylactic acid (PLLA). Such scaffold has an outer sheath formed by electrospun nanofibers without a preferential orientation, which coats a certain number of clusters of previously implemented twisted nanofibers (yarns). The applicability limitations of this construct in the field of tissue engineering of tendinous and/or ligamentous and/or muscular and/or nervous tissue appear evident.

First of all, in fact, if for the spinning of the outer sheath a drum ground collector is used, around which the clusters of twisted nanofibers (yarns), which will constitute the complete scaffold, are fastened manually, such technological solution does not allow good compaction of such clusters of twisted nanofibers (yarns) in the complete scaffold, by limiting evidently the final mechanical properties thereof; moreover, this approach requires a subsequent removal of the ground collector from the final construct, a procedure which could compromise the integrity of the scaffold itself. At last, the technological solution adopted by Dawei at al. allows to coat a limited number of clusters of twisted nanofibers (yarns) electrospun with the outer sheath: upon increasing the number of clusters of twisted nanofibers (yarns) and/or the diameter of the clusters of twisted nanofibers (yarns), or in case upon reducing the diameter of the drum collector, there is great risk that the ground effect of the collector is drastically weakened, making impossible the deposition of the electrospun fibers constituting the outer sheath.

Furthermore, in literature up to know no valid solutions are provided capable of being able to effectively suture and/or fix such clusters of twisted nanofibers (yarns) and/or clusters of axially aligned nanofibers (bundles) to the interface with the damaged tissue and/or with muscular and/or bone interface.

Jackson et al. (WO 2010/062297 A1) describe a support (scaffold), for the regeneration di some tissues such as the nervous one, obtained through the electrospinning technology, constituted by a porous sheath of randomly arranged nanofibers (random), which includes an inner portion constituted by single nanofibers, obtained through electrospinning technology too, axially aligned with respect to the scaffold itself. According to the description of the authors, in order to electrospin the scaffold two synchronous rotating cylinders, and having a gap therebetween (gap), are used as ground collectors. During the initial phase of the electrospinning process the nanofibers, attracted by the two ground collectors, adhere on the two faced sides of the rollers, filling-up the gap between the rollers themselves and by aligning. Once coated the inner faces of the rollers with aligned nanofibers, by continuing the process, the nanofibers arrange randomly, by constituting a sheath which coats the aligned nanofibers (attracted by the axial area of the two rollers). At the end of the process a porous scaffold is obtained, constituted by single nanofibers axially aligned inside thereof, coated by a sheath of nanofibers arranged randomly. The first limitation lies in the impossibility of being able to modulate the final length of the scaffold. In fact, it is known that upon increasing the distance between the rollers, progressively the ground effect between the two rollers decreases until it disappears completely, by allowing the nanofibers to deposit only on the two rollers separately, by making impossible the production of the scaffold and/or the alignment of the nanofibers. Moreover, morphologically such scaffold has a quite different structure with respect to the nervous tissue, which is characterized by a complex, compact, hierarchical and multiscale fibrous structure, organized in substructures which join in different levels to create the complete tissue. In the multiscale hierarchical structure of the nerves, the fundamental elements of such tissues lie in the mielinized neuronal axons. The neuronal axons align and join in groups, the neuronal fascicles; groups of neuronal fascicles are joined and compacted with one another by a fibrous sleeve called epineurium to obtain the complete nerve. Moreover, in some nerves, single sub-groups of neuronal fascicles are joined therebetween by sleeves called perineurium, and in turn, these structures are tied up together by the epineurium sleeve. All the just mentioned structures are not reproduced morphologically by the scaffold proposed by the authors which, on the contrary, appears with nor hierarchical nor multiscale three-dimensional structure. Such lack in hierarchical organization in the scaffold proposed by the authors further compromises the mechanical properties of the scaffold itself which, since it includes inside thereof only aligned nanofibers, but having no organization in substructures and no level of compaction, are not able to offer satisfying mechanical resistance to support the physiological loads thereto such tissues are in vivo subjected.

Sensini et al. (Biofabrication. 2017 Mar 8;9(1):015025) describe a method for producing, through the electrospinning technology, clusters of axially aligned nanofibers (bundles). For their implementation, polymeric nanofibers are electrospun on a drum collector, rotating at high speed. At the end of the electrospinning process circumferential strips of membrane of nanofibers are cut on the drum, and they are rolled up by forming the clusters of axially aligned nanofibers (bundles) (each one having diameter of about 500 micrometers) which are then cut and removed from the drum. However, the authors show these clusters of axially aligned nanofibers (bundles) as scaffolds which mimic the morphology and mechanical properties of single tendinous fascicles of collagen, without proposing any method in order to produce a multiscale hierarchical scaffold capable of reproducing a complete tendon.

What above said shows a huge lack in the literature for reproducing multiscale hierarchical scaffolds capable of reproducing in each element the hierarchical structure of tendons and/or ligaments and/or muscles and/or nerves.

The state of art as above summarized shows then the need for providing new supports and methods for their production not having the disadvantages of those of state of art.

### Summary of the invention

The scope of the invention is defined by the claims. Any references in the description to methods of treatment refer to the products of the present invention for use in a method for treatment.

The inventors have succeeded in obtaining a support (*scaffold*) allowing to replace and/or reconstruct the tendinous and/or ligamentous and/or muscular and/or nervous tissue through a construct with hierarchical and multiscale three-dimensional structure (multiscale hierarchical scaffold) capable of reproducing the mechanical, morphological and physiological features of tendons and ligaments and/or muscles and/or nerves.

In the present description under construct and/or multiscale hierarchical scaffold each device or structure is meant, constituted by sub-structures, with different dimensional scales, mutually joining in a hierarchical order. This configuration is typical of the connective tissues such as tendons and/or ligaments, and also of the muscles and nerves. In fact, the tendinous and/or ligamentous and/or muscular and/or nervous tissues are constituted by a multiscale hierarchical structure constituted by different features (enlisted hereinafter starting from the molecular level as far as the whole tendon and/or ligament and/or muscle and/or nerve).

In the multiscale hierarchical structure of tendons and/or ligaments, the fundamental elements of such tissues lie in the tropocollagen molecules which are joined with one another by producing a collagen fibril; the collagen fibrils are aligned in different groups, called fascicles; groups of fascicles are joined and compacted with each other by a sleeve of collagen fibrils, called epitenon/epiligament, to form the whole tendon or ligament. Moreover in some tendons and/or ligaments, single sub-groups of fascicles are joined therebetween by sleeves called endotenon/endoligament and, in turn, these structures are tied up together by the epitenon/epiligament sleeve.

The natural multiscale hierarchical structure of tendons and/or ligaments is perfectly mimiked by the multiscale hierarchical scaffold the present invention relates to: the electrospun nanofibers (fibers having diameter of nanometers, comparable to the collagen fibrils of tendons and/or ligaments) consist of polymer macromolecules (at a dimensional scale similar to the tropocollagen); a plurality of electrospun nanofibers are aligned to form a cluster of axially aligned nanofibers (bundle) and/or twisted to form a cluster of twisted nanofibers (yarn) (similar to the fascicle of tendons and/or ligaments); the plurality of clusters of axially aligned nanofibers (bundles), and/or the plurality of clusters of twisted nanofibers (yarns), are joined and compacted with each other by an electrospun sheath of nanofibers (imitating the epitenon/epiligament sleeve of tendons and/or ligaments) which forms the multiscale hierarchical scaffold the present invention relates to. Moreover, several multiscale hierarchical scaffolds, the invention relates to, can be joined, in turn, therebetween in an additional hierarchical level, by an additional electrospun sheath produced similarly to the previous one. In this case the sheath coating the single multiscale hierarchical scaffolds will simulate the endotenon/endoligament sleeves of tendons and/or ligaments, whereas the outer sheath joining it will simulate the epitenon/epiligament of tendons and/or ligaments.

In the hierarchical and multiscale structure of the muscles, the fundamental elements of such tissues lie in the molecules of actin and myosin, joined with each other by producing filamentous structures called muscle fibres. The muscle fibres align and join in groups, the muscle fascicles; groups of muscle fascicles are joined and compacted with each other by a fibrous sleeve called epimysium, by forming the complete muscle. Moreover, in some muscles, sub-groups of muscle fascicles are joined therebetween by sleeves called perimysium, and in turn these structures are tied up together by epimysium.

The natural multiscale hierarchical structure of the muscles is perfectly mimiked by the multiscale hierarchical scaffold the present invention relates to: the polymer macromolecules (at a dimensional scale comparable to actin and myosin of the muscles), form the electrospun nanofibers (comparable to the muscular fibrils); a plurality of electrospun nanofibers are aligned to form a cluster of axially aligned nanofibers (bundle), and/or twisted to form a cluster of twisted nanofibers (yarn) (similar to the muscle fascicle); the plurality of clusters of axially aligned nanofibers (bundles), and/or the plurality of clusters of twisted nanofibers (yarns), are joined and compacted with each other by an electrospun sheath of nanofibers (imitating the epimysium sleeve) which forms the multiscale hierarchical scaffold, the present invention relates to. Moreover, several multiscale hierarchical scaffolds the invention relates to, can be joined, in turn, therebetween in an additional hierarchical level by an additional electrospun sheath produced similarly to the previous one. In this case the sheath coating the single multiscale hierarchical scaffolds, will simulate the perimysium sleeves of the muscles, whereas the outer sheath joining them will simulate the epimysium sleeve of the muscle tissue.

In the multiscale structure of the nerves, all fundamental elements of such tissues lie in the mielinized neuron axons. The neuron axons align and join in groups, the neuron fascicles; groups of neuron fascicles are joined and compacted with each other by a fibrous sleeve called epineurium in order to obtain the complete nerve. Moreover, in some nerves, single sub-groups of neuron fascicles are joined therebetween by sleeves called perineurium, and in turn, these structures are tied up together by the epineurium sleeve.

The natural multiscale hierarchical structure of the nerves is perfectly mimiked by the multiscale hierarchical scaffold the present invention relates to: a plurality of electrospun nanofibers (for guiding the growth of the neuron sprouts and cells of Schwann) are aligned and grouped in clusters of axially aligned nanofibers (bundles) and/or twisted in clusters of nanofibers (yarns) (similar to the fascicles of the nerves); the plurality of clusters of nanofibers axially aligned (bundles) and/or the plurality of clusters of twisted nanofibers (yarns), are joined and compacted with each other by an electrospun sheath of nanofibers (imitating the epineurium sleeve of the nerves) which forms the multiscale hierarchical scaffold, the present invention relates to, Moreover, several multiscale hierarchical scaffolds, the invention relates to, can be joined, in turn, therebetween in an additional hierarchical level by an additional electrospun sheath produced similarly to the previous one, In this case the sheath coating the single multiscale hierarchical scaffolds will simulate the perineurium sleeves of the nerves, whereas the outer sheath joining them will simulate the epineurium sleeve of nervous tissue.

The process used for producing such multiscale hierarchical scaffold allows to produce an outer electrospun sheath, which can also be used to compact sub-groups of clusters of axially aligned nanofibers (bundles) and/or of clusters of twisted nanofibers (yarns) inside the multiscale hierarchical scaffold, without using ground collectors inside the body of the multiscale hierarchical scaffold. Moreover, such sheath provides protection and mechanical compaction of the clusters of axially aligned nanofibers (bundles) and/or clusters of twisted nanofibers (yarns) inside thereof, by allowing the passage of cells at the same time.

The multiscale hierarchical scaffold obtained by the inventors succeeds in repeating various aggregation levels of the tendinous /ligamentous tissue, from collagen fibrils to the tendinous/ligamentous fascicles, until reaching the complete tendon/ligament. Moreover, the inventors have succeeded in developing a process in order to be able to e!ectrospin a sheath of nanofibers on the surface of the clusters of axially aligned nanofibers (bundles) and/or clusters of twisted nanofibers (yarns), similar by morphology to the sleeve coating the tendons/ligaments (epitenon), in case by succeeding in coating also sub-groups thereof (endotenon), so as to allow the compaction of the clusters of axially aligned nanofibers (bundles) and/or clusters of twisted nanofibers (yarns) and to favour the mechanical resistance thereof on one side, but even to allow the passage of the cells through the sheath and the colonization of the clusters of axially aligned nanofibers (bundles) and/or clusters of twisted nanofibers (yarns).

An additional advantage of the herein described multiscale hierarchical scaffold lies in the possibility of including inside thereof any number of clusters of axially aligned nanofibers (bundles) and/or clusters of twisted nanofibers (yarns) having any diameter, compacted and joined through a porous electrospun sheath capable of guaranteeing on one side protection to the clusters of axially aligned nanofibers (bundles) and/or clusters of twisted nanofibers (yarns) inside thereof, on the other side the passage of the cells therethrough with the purpose of being able to deposit on the clusters of axially aligned nanofibers (bundles) and/or clusters of twisted nanofibers (yarns) and to reconstruct the tendinous/ligamentous and/or muscular and/or nervous extracellular matrix. The electrospun sheath is produced without using collectors inside to the multiscale hierarchical scaffold and it is capable of compacting and in case twisting (*twisting*) the clusters of axially aligned nanofibers (bundles) and/or clusters of twisted nanofibers (yarns) inside thereof. Such sheath can also be used to coat sub-groups of clusters of axially aligned nanofibers (bundles) and/or clusters of twisted nanofibers (yarns) inside the multiscale hierarchical scaffold by further increasing the mechanical properties and the aggregation hierarchical level.

Therefore, firstly the present invention relates to a multiscale hierarchical scaffold for the replacement and/or repair and/or regeneration and/or reconstruction and/or simulation of a tissue, in particular of the tendinous and/or ligamentous and/or muscular and/or nervous tissue comprising:
- a plurality of clusters obtained by electrospinning, each one consisting of nanofibers, wherein said plurality of clusters are arranged in order to form one single group;
- a porous sheath obtained by electrospinning consisting of nanofibers, wherein said sheath externally coats and compacts said plurality of clusters by keeping them aligned with each other.

Such sheath is also capable of joining groups of multiscale hierarchical scaffolds with each other, by increasing the aggregation hierarchical level.

Said sheath then succeeds in compacting the clusters of axially aligned nanofibers (bundles) and/or twisted nanofibers (yarns) by keeping them aligned with each other at the same time, without the need of having to interlace them, to twist them and without the possible use of compacting fillers to obtain the same effect, differently from the case of the previously developed constructs, by consequently guaranteeing mechanical properties similar to those typical of the tendinous and/or ligamentous and/or muscular and/or nervous body tissue, allowing at the same time to mimic absolutely fidelic the hierarchical organization of the tendinous and/or ligamentous and/or muscular and/or nervous tissue itself.

Such feature results to be substantially important for a scaffold devised for the regeneration of the tendinous and/or ligamentous and/or muscular and/or nervous tissue: in fact, by varying the morphological structure of the scaffold from that of the native tissue, the risk that scar tissue arises is very strong, with consequent depletion of the final mechanical and morphological properties of the regenerated tissue. Said sheath further allows the passage of the cells which should colonize the whole multiscale hierarchical structure; moreover, it allows the correct vascularization of the cell component and the removal of the waste components produced by the cells during the reconstruction of the subject tissues. The resulting scaffold has high mechanical features (resistance and stiffness), of the same order of magnitude of the tendinous and/or ligamentous and/or muscular and/or nervous human tissues.

Secondly, the present invention relates to a process for preparing a multiscale hierarchical scaffold for the replacement, repair or reconstruction of a tissue, in particular of the tendinous and/or ligamentous and/or muscular and/or nervous tissue, comprising the following steps:
a) preparing by electrospinning a plurality of clusters of axially aligned nanofibers (bundles) and/or clusters of twisted nanofibers (yarns);
b) electrospinning nanofibers so as to coat said plurality of clusters of axially aligned nanofibers (bundles) and/or clusters of twisted nanofibers (yarns) with a porous sheath which consists of nanofibers so as to provide an external coating and to compact the plurality of clusters of axially aligned nanofibers (bundles) and/or clusters of twisted nanofibers (yarns) prepared according to step a).

In particular according to an embodiment such process provides the following steps:
a) preparing by electrospinning a plurality of clusters each one consisting of axially aligned nanofibers (bundles) and/or clusters of twisted nanofibers (yarns);
b) positioning said plurality of clusters of axially aligned nanofibers (bundles) and/or clusters of twisted nanofibers (yarns) prepared at step a) so as to form one single group;
c) clamping the group of clusters of axially aligned nanofibers (bundles) and/or clusters of twisted nanofibers (yarns) obtained at step b) on a grip capable of axially rigidly rotating and in line, thus by keeping the clamped group of clusters of axially aligned nanofibers (bundles) and/or clusters of twisted nanofibers (yarns) in suitable position for the coating process with electrospun sheath;
d) implementing an outer sheath of the group of clusters of axially aligned nanofibers (bundles) and/or clusters of twisted nanofibers (yarns) clamped at step c) by electrospinning, in particular by controlling the rotation parameters of the clamped group of clusters of axially aligned nanofibers (bundles) and/or clusters of twisted nanofibers (yarns), the geometrical parameters of the setup, and process parameters.

The sheath which is produced has the effect to compact the clusters of axially aligned nanofibers (bundles) and/or twisted nanofibers (yarns), by reducing to minimum the gaps between the different clusters and thus by reducing to minimum the global section of the multiscale hierarchical scaffold. Such effect allows to obtain a construct capable of expressing mechanical properties comparable to those of the natural tissues which it has to mimic, and homogeneous for the whole section thereof.

By replacing the group of clusters of axially aligned nanofibers (bundles) and/or clusters of twisted nanofibers (yarns), according to step b), with a group of multiscale hierarchical scaffolds, and by repeating the steps c) and d), it will be further possible to obtain a multiscale hierarchical scaffold with a further increased aggregation hierarchical level, as in turn constituted by a plurality of multiscale hierarchical scaffolds.

Thirdly, the invention relates to a new electrospun construct called ring-like cluster of nanofibers (ring bundle). Such construct is configurated morphologically like a closed ring, consisting of randomly arranged nanofibers (random) and/or with an alignment degree along the axial direction of the ring. Such ring-like clusters of nanofibers (ring bundles) will be not necessarily used in the original circular shape, but they could be stretched according to a direction to obtain a closed elongated shape. In particular such ring-like clusters of nanofibers (ring bundle), if used singularly and/or placed side by side to a plurality of ring-like clusters of nanofibers similar to this one, could be used to construct multiscale hierarchical electrospun scaffolds similar to tendons and/or ligaments and/or muscles and/or nerves. In particular if replaced, the clusters of axially aligned nanofibers (bundles) and/or clusters of twisted nanofibers (yarns), at step a), with a single ring-like cluster of nanofibers (ring bundle) and/or with a plurality of ring-like clusters of nanofibers (ring bundles), and by repeating the procedure for the production of the sheath from step b) to step d), the electrospun sheath will compact the central area of the ring-like clusters of electrospun nanofibers (ring bundles), by providing thereto an elongated shape, and by generating loops at the end of the multiscale hierarchical scaffold. Such loops for example will result to be useful to suturing and/or fixing the so-obtained multiscale hierarchical scaffold, to the interface with the tendon and/or ligament and/or muscle and/or nerve and/or bone of interest. Moreover, such ring-like clusters of nanofibers (ring bundles) could be arranged inside the multiscale hierarchical scaffolds, both axially aligned with each other and/or twisted (twisted) with each other and/or singularly twisted and axially placed side by side to each other and/or twisted (twisted) singularly and in turn twisted (twisted) with each other and/or placed in the same scaffold together with one or more clusters of axially aligned nanofibers (bundles) and/or one or more clusters of twisted nanofibers (yarns).

Such ring-like clusters of electrospun nanofibers (ring bundles) in turn could consist of randomly arranged (random) and/or axially aligned and/or twisted nanofibers.

Other advantages, features and use modes of the present invention will result evident from the following detailed description of some embodiments, shown by way of example and not for limitative purposes.

### Brief description of the figures

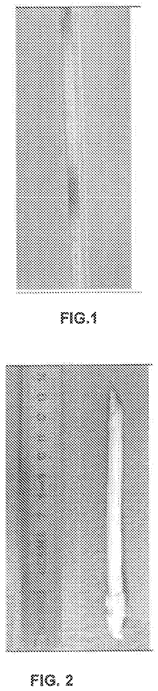

- Figure 1 is a picture of one single cluster of axially aligned nanofibers (bundle) of the multiscale hierarchical scaffold according to a preferred embodiment of the present invention.
- Figure 2 is a picture showing the multiscale hierarchical scaffold fully, which consists of a plurality of clusters of axially aligned nanofibers (bundles) (Figure 1) kept together by an outer sheath of randomly arranged fibers according to a preferred embodiment of the present invention.

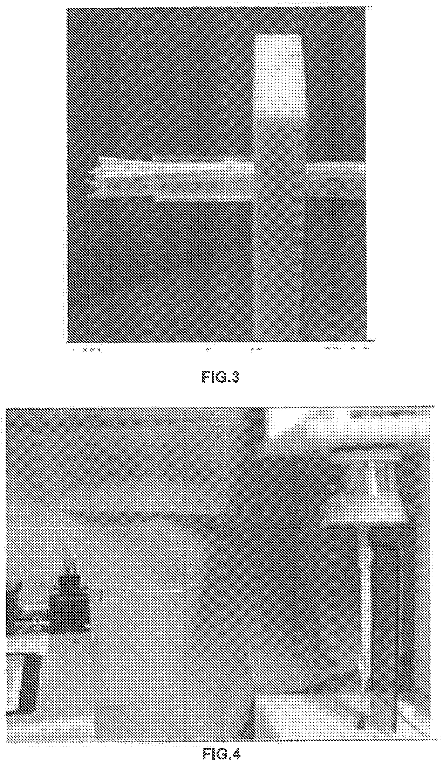

- Figure 3 is a picture of the experimental set-up allowing to fix the clusters of axially aligned nanofibers (bundles) and/or clusters of twisted nanofibers (yarns) parallelly to each other and one placed side by side in the phase preceding the spinning of the sheath according to a preferred embodiment of the present invention.
- Figure 4 is a picture of the experimental set-up allowing to coat the clusters of axially aligned nanofibers (bundles) and/or clusters of twisted nanofibers (yarns) with an outer sheath of nanofibers according to a preferred embodiment of the present invention.

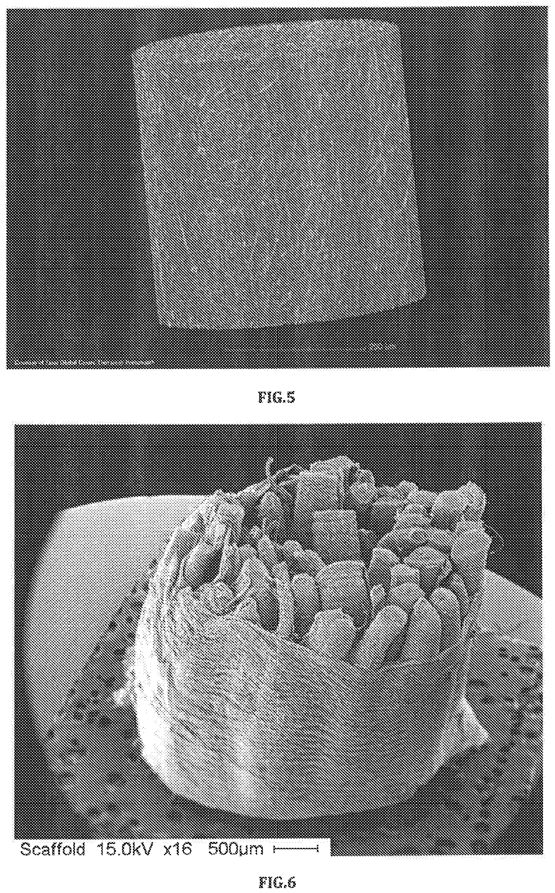

- Figure 5 is a tomographic image of the nanofibers of one single cluster of axially aligned nanofibers (bundle) in poly(L)lactic acid (PLLA).
- Figure 6 is a SEM image of the section of a multiscale hierarchical scaffold in PLLA constituted by 100 clusters of axially aligned nanofibers (bundles) and with electrospun outer sheath.

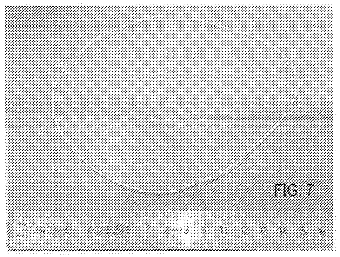

- Figure 7 is a picture of one single ring-like cluster of nanofibers (ring bundle) according to a preferred embodiment of the present invention.

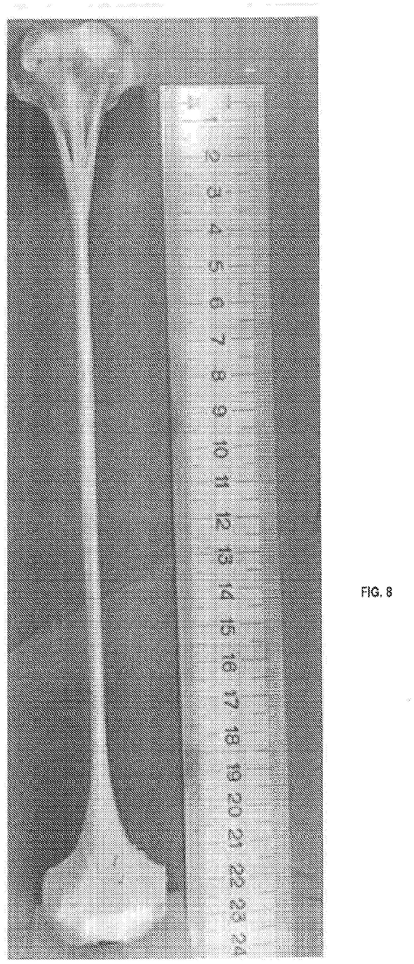

- Figure 8 is a picture showing the multiscale hierarchical scaffold fully, which consists of a plurality of ring-like clusters of nanofibers (ring bundles) (Figure 7) kept together by an outer sheath of randomly arranged nanofibers according to a preferred embodiment of the present invention, by showing the loops for suturing and/or fixing at their own ends.

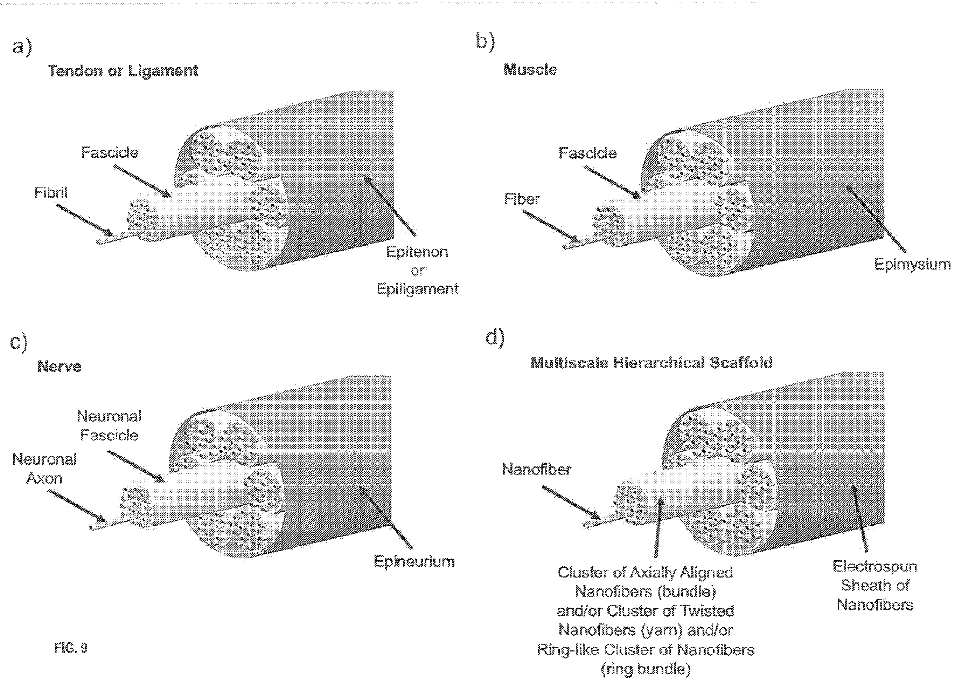

- Figure 9 is a schematic representation of the multiscale hierarchical levels of aggregation of the tendinous and/or ligamentous, muscular and nervous tissue, compared with the hierarchical structure of the multiscale hierarchical scaffold the present invention relates to. In particular: a) Multiscale hierarchical structure of tendons and/or ligaments; b) Multiscale hierarchical structure of muscles; c) Multiscale hierarchical structure of nerves; d) Multiscale hierarchical scaffold structure.

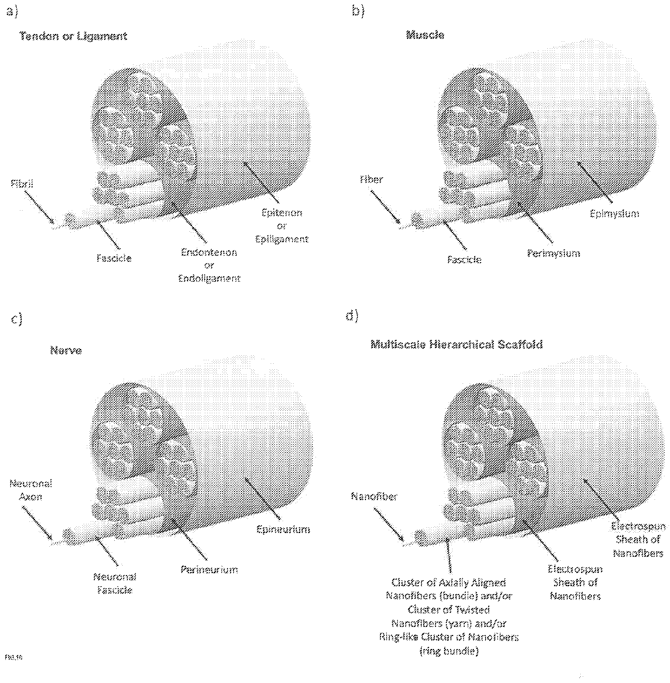

- Figure 10 is a schematic representation of the multiscale hierarchical levels of aggregation of the tendinous and/or ligamentous, muscular and nervous tissue according thereto further down hierarchical units of the same tissue join together by increasing the multiscale hierarchical level of the same. Such levels are compared with the hierarchical structure of the multiscale hierarchical scaffold, the present invention relates to, according to the embodiment wherein groups of multiscale hierarchical scaffolds are joined together by an additional sheath of electrospun nanofibers, by forming a multiscale hierarchical scaffold with a higher level of hierarchical organization. In particular: a) Multiscale hierarchical structure of tendons and/or ligaments; b) Multiscale hierarchical structure of muscles; c) Multiscale hierarchical structure of nerves; d) Multiscale hierarchical scaffold structure in turn constituted by groups of multiscale hierarchical scaffolds.

### Detailed description of the invention

The present invention relates to a multiscale hierarchical scaffold, to the processes for the production thereof and the uses thereof.

In the present description under hierarchical and multiscale it is meant: each device or structure, constituted by sub-structures, having different dimensional scales, which join in a hierarchical order.

In the present description under the expression "support or a multiscale hierarchical scaffold" it is meant: a porous anisotropic three-dimensional construct constituted by biomaterials assembled at morphological level at different levels of dimensional scale (from nanometric to micrometric and millimetric), hierarchically organized in a multiscale structure (as defined previously), to mimic as exactly as possible the extracellular matrix of the tissue which one wants to reconstruct in its native state. The scaffolds are typically designed to perform the following functions: (i) promoting the cell-biomaterial interaction, the cell adhesion and the cell proliferation, (ii) allowing the transportation of oxygen, carbon dioxide and nutrients, (iii) if bioresorbable, biodegrading at a speed approximating the tissue regeneration rate under the culture conditions of interest, (iv) not causing *in vivo* inflammation or toxicity and (v) having mechanical properties similar to the tissue which one wants to reconstruct.

Even a not bioresorbable material could be selected, but in this case it should not cause *in vivo* inflammation or toxicity and have mechanical and morphological properties similar to the tissue which one wants to reconstruct and/or simulate and/or replace.

In the present description under "cluster of axially aligned nanofibers (bundle)" an electrospun structure is meant, with variable extension and/or section, consisting of nanofibers which arrange with a level of alignment according to the axis of the cluster itself, that is along the greater development direction of these constructs. In the present description under "cluster of twisted nanofibers (yarn)" an electrospun structure is meant, with variable extension and/or section, consisting of nanofibers which arrange with a level of twisting according to the axis of the yarn itself, that is along the longitudinal direction of these constructs.

In the present description under "ring-like cluster of nanofibers (ring bundle)" a ring-shaped electrospun structure is meant, with variable extension and section, consisting of nanofibers, with a level of alignment according to the axis of the ring-like duster or ring bundle itself.

in the present description under the expression "forming one single group" the fact of forming one single cluster is meant.

In an additional embodiment the nanofibers constituting the ring-like cluster could be arranged randomly (*random*) and/or in a twisted way (twisted) and/or with a level of axial alignment inside the body of the ring-like cluster or ring bundle itself. The multiscale hierarchical scaffold according to the present invention further comprises a porous sheath obtained by electrospinning which coats externally the clusters of axially aligned nanofibers (bundles) and/or clusters of twisted nanofibers (yarns) and/or ring-like clusters of nanofibers (ring bundles), obtained by electrospinning. Said outer sheath (casing) is constituted by nanofibers too. The porosity of the sheath is produced by the superimposition of layers of continuous nanofibers which deposit in the process period of time on a same plane, by forming a three-dimensional structure like the one of a tissue-non-tissue. The porosity of the sheath is then interconnected, in the sense that the pores put into communication the outer layer of the sheath of fibers with the innermost layer, in contact with the clusters of axially aligned nanofibers (bundles) and/or clusters of twisted nanofibers (yarns) and/or ring-like clusters of nanofibers (ring bundles) and/or sub-groups of multiscale hierarchical scaffolds. The interconnection between the pores allows the cells to filtrate through the outer sheath to reach the inner layers of the multiscale hierarchical scaffold.

The nanofibers of the sheath could be arranged randomly and/or with a level of axial alignment and/or level of peripheral alignment.

The average diameter of the nanofibers constituting the multiscale hierarchical scaffold could be comprised between 10 and 100000 nm.

The length of the multiscale hierarchical scaffold could be comprised between 10 and 1000 mm, in particular between 10 and 500 mm, preferably 20 and 200 mm and it will have an average diameter comprised between 1 and 100 mm, preferably comprised between 2 and 50 mm. According to an embodiment the multiscale hierarchical scaffold will be made of bioresorbable or biostable and/or inert material.

Examples of bioresorbable or biostable materials of synthetic origin are polyesters, polyurethanes, polyamides, polyolefins, fluorinated polymers and copolymers thereof. Examples of bioresorbable or biostable materials of natural origin are polysaccharides, proteins, polyesters, polypeptides and copolymers thereof. Preferred examples of materials for preparing the nanofibers are poly-(L)-lactic acid (PLLA) and/or collagen and/or nylon 6,6, also other biocompatible polyamides known to the person skilled in the art could be used.

According to an embodiment the multiscale hierarchical scaffold and/or the nanofibers it consists of, could be loaded and/or functionalized with components of organic and/or inorganic nature which play a biological action and/or change in the chemical-physical and/or mechanical properties of the tissue wherein the multiscale hierarchical scaffold could be used. For example components of organic and/or inorganic nature which could be used are drugs, growth factors, antibacterial agents, peptides, hydroxyapatites, phosphates, bio-glasses, metal oxides, graphene, carbon nanotubes.

In an embodiment of the nanofibers constituting the multiscale hierarchical scaffold and/or the sheath and/or the clusters of axially aligned nanofibers (bundles) and/or the clusters of twisted nanofibers (yarns) and/or the ring-like clusters of nanofibers (ring bundles) they could be, from the point of view of morphology, classic nanofibers constituted by one single phase (made of one single material and/or by a mixture of materials and/or loaded and/or functionalized materials) and/or nanofibers constituted by two or more phases (for example core-shell nanofibers, wherein, under core-shell, nanofibers are meant made of different materials between central portion and outer portion of the nanofiber itself) and/or hollow-shell nanofibers (wherein, under hollow-shell, nanofibers are meant constituted by an inner central cavity along the axis of the nanofibers themselves) and/or porous nanofibers (under porous nanofibers nanofibers are meant having pores along their surface and/or in their inner volume).

The procedures for loading and/or functionalizing and/or producing nanofibers with different morphology are known to the person skilled in the art.

The nanofibers constituting the single clusters of axially aligned nanofibers (bundles) and/or clusters of twisted nanofibers (yarns) and/or ring-like clusters of nanofibers (ring bundles) and/or the sheath and/or the sheaths will have an average diameter comprised between 10 and 10000 nm, preferably comprised between 200 and 1000 nm, and in particular between 300 and 1000 nm, whereas the average diameter of the clusters of axially aligned nanofibers (bundles) and/or clusters of twisted nanofibers (yarns) and/or ring-like clusters of nanofibers (ring bundles) could be comprised between 10 and 10000 µm, in particular comprised between 20 and 10000 µm, preferably between 500 and 650 µm. The number of clusters of axially aligned nanofibers (bundles) and/or clusters of twisted nanofibers (yarns) and/or ring-like clusters of nanofibers (ring bundles) will be for example comprised between 2 and 1000, preferably between 40 and 200, for example 100.

The multiscale hierarchical scaffolds according to the present invention advantageously will have a value of mechanical resistance comprised between 10 and 5000 N preferably between 200 and 500 N and/or an elastic modulus comprised between 20 and 100000 MPa, preferably of about 30-20000 MPa. Such mechanical features were measured by means of monoaxial tensile test with capstan grips (*capstan grips*) and by cementing the ends as described in the examples.

The present invention further relates to a process for preparing a multiscale hierarchical scaffold for the replacement and/or repair and/or regeneration and/or reconstruction and/or simulation of a tissue, in particular of the tendinous and/or ligamentous and/or muscular and/or nervous tissue comprising the following steps:
a) preparing by electrospinning a plurality of clusters of axially aligned nanofibers (bundles) and/or of clusters of twisted nanofibers (yarns)
b) electrospinning nanofibers so as to coat a plurality of said clusters of axially aligned nanofibers (bundles) and/or clusters of twisted nanofibers (yarns) with a porous sheath consisting of nanofibers so as to provide an external coating and to compact the plurality of clusters of axially aligned nanofibers (bundles) and/or clusters of twisted nanofibers (yarns) prepared according to step a) by obtaining a support or a multiscale hierarchical scaffold;
c) electrospinning nanofibers so as to coat a plurality of said supports or multiscale hierarchical scaffolds according to step b) with an additional porous sheath consisting of nanofibers so as to provide an external coating and to compact the plurality of clusters of axially aligned nanofibers (bundles) and/or clusters of twisted nanofibers (yarns) and/or multiscale hierarchical scaffolds according to step b).

An embodiment of the present invention is a process for preparing ring-like clusters of nanofibers (ring bundles) comprising the following steps:
a) electrospinning on a ground collector shaped like a drum rotating at different speeds according to the wished alignment level a plurality of nanofibers;
b) cutting peripheral strips of membrane of nanofibers obtained by the electrospinning according to step a);
c) rolling-up the peripheral strips of membrane of nanofibers obtained according to the step b) according to the drum axis;
d) pulling out the drum the ring-like clusters of nanofibers (ring bundles) obtained according to step c).

In particular the process for preparing multiscale hierarchical scaffolds with ring-like clusters of nanofibers (ring bundles) will provide the following steps:
a) preparing by electrospinning a plurality of clusters of axially aligned nanofibers (bundles) and/or clusters of twisted nanofibers (yarns) and/or ring-like clusters of nanofibers (ring bundles);
b) positioning said plurality of clusters of axially aligned nanofibers (bundles) and/or clusters of twisted nanofibers (yarns) and/or clusters of ring-like nanofibers (ring bundles) prepared according to step a) so as to form one single group of clusters of axially aligned nanofibers (bundles) and/or clusters of twisted nanofibers (yarns) and/or ring-like clusters of nanofibers (ring bundles);
c) clamping the group obtained according to step b) on grip capable of axially rotating rigidly and in line, thus keeping the group of clusters of nanofibers and/or axially aligned nanofibers (bundles) and/or clusters of twisted nanofibers (yarns) and/or ring-like clusters of nanofibers (ring bundles), clamped in a suitable position for the coating process with electrospun sheath;
d) implementing an outer sheath on the group of clusters of axially aligned nanofibers (bundles) and/or clusters of twisted nanofibers (yarns) and/or ring-like clusters of nanofibers (ring bundles) clamped at step c) by electrospinning, in particular by controlling the rotation parameters of the clamped group of clusters of axially aligned nanofibers (bundles) and/or clusters of twisted nanofibers (yarns) and/or ring-like clusters of nanofibers (ring bundles), the geometrical parameters of the setup, and process parameters, by obtaining a multiscale hierarchical structure or scaffold.
e) implementing an additional outer sheath on the group of structures or multiscale hierarchical scaffolds obtained according to step d) and clamped as according to step c) by electrospinning, in particular by controlling the rotation parameters of the clamped group of structures or multiscale hierarchical scaffolds, the geometrical parameters of the setup, and process parameters.

According to an embodiment the nanofibers of the clusters of axially aligned nanofibers (bundles) and/or clusters of twisted nanofibers (yarns) and/or ring-like clusters of nanofibers (ring bundles) and/or of the sheath could be prepared by electrospinning a solution of PLLA dissolved in suitable solvent, for example in dichloromethane (DCM) and N,N-dimethylformamide (DMF). The solution could be prepared for example with 10-30% (weight/volume) of PLLA for example in 65/35 (volume/volume) (DCM/DMF).

According to an embodiment the nanofibers of the clusters of axially aligned nanofibers (bundles) and/or clusters of twisted nanofibers (yarns) and/or ring-like clusters of nanofibers (ring bundles) and/or of the sheath could be prepared by electrospinning a solution of nylon 6,6 dissolved in suitable solvent, for example in trifluoroacetic acid (TFA) and acetone (AC). The solution could be prepared with 10-30% (weight/volume) of nylon 6,6 for example in 50/50 (volume/volume) (TFA/AC).

According to an embodiment the spinning conditions of the clusters of axially aligned nanofibers (bundles) and/or of the clusters of twisted nanofibers (yarns) and/or of the ring-like clusters of nanofibers (ring bundles) provide the application of an electrical field having a voltage comprised between 10 and 30 kV, preferably 18 kV for an electrospinning time period of at least 5 min, and in particular at least 15 min, preferably one hour, by depositing the fibres on a collector rotating at high speed allowing an alignment degree of the nanofibers, The nanofibers deposited on the collector are subsequently collected together to form clusters of axially aligned nanofibers (bundle) and/or clusters of twisted nanofibers (yarn) and/or a ring-like clusters of nanofibers (ring bundles) with an alignment degree.

According to an embodiment the electrospinning conditions on the rotating machine for the production of the outer sheath of nanofibers provide the application of an electrical field having a voltage comprised between 10 and 30 kV, for an electrospinning time period of at least 2 hours, preferably 3 hours.

Advantageously for preparing the sheath the following process parameters will be applied:
- distance between the group of clusters and the flat collector smaller than 5 mm;
- a rotation speed of the group of clusters of about 20-25 rpm;
- stillness periods of the group of clusters of about 3-5 min;
- rotation periods of the group of clusters 1-2 min.

The process for preparing the multiscale hierarchical scaffold according to the present invention has important advantages, in particular the development of the sheath around the clusters of axially aligned nanofibers (bundles) and/or clusters of twisted nanofibers (yarns) and/or ring-like clusters of nanofibers (ring bundles), which is produced without inserting anything in the body of a multiscale hierarchical scaffold to guide the deposition of fibers.

Such result is obtained by modulating the shape, the sizes and the position of the collector placed nearby, but not in contact with the group of clusters of axially aligned nanofibers (bundles) and/or clusters of twisted nanofibers (yarns) and/or ring-like clusters of nanofibers (ring bundles) to be coated and modulating the rotation and stasis time periods of the same, for example it could be positioned at a distance comprised between 1 and 50 mm.

Making this way, during the stillness time periods the clusters of axially aligned nanofibers (bundles) and/or clusters of twisted nanofibers (yarns) and/or ring-like clusters of nanofibers (ring bundles) will be surrounded by a layer of nanofibers which will have its own ends deposited on the collector. By putting in rotation the group of clusters, the detachment of one end of the layer of nanofibers from the flat collector will take place, by wrapping the group of clusters of nanofibers, favouring the compaction and the pretensioning. Once the whole layer of nanofibers is rolled up around the group of clusters of nanofibers, the end still attached to the flat collector will detach, in turn. Once the detachment is completed, thanks to the combined action of rotation and electrostatic field even the remaining portion of the layer of nanofibers will deposit on the surface of the clusters of nanofibers. The repetition of this process progressively leads to the compaction with consequent reduction in the section of the group of clusters of nanofibers and to the complete formation of the sheath to obtain the complete multiscale hierarchical scaffold.

Similarly, by repeating the process for producing the sheath of nanofibers on a plurality of multiscale hierarchical scaffolds, produced as previously described, and joined together to form one single group, it will be possible to obtain a multiscale hierarchical scaffold in turn constituted by a plurality of multiscale hierarchical scaffolds, all of them coated and compacted by an electrospun sheath of nanofibers.

According to an embodiment, the grips for fixing the clusters of axially aligned nanofibers (bundles) and/or twisted nanofibers (yarns) and/or ring-like clusters of nanofibers (ring bundles), capable of rotating rigidly and in line thus keeping the group of clusters of nanofibers, clamped in suitable position for the coating process with electrospun sheath, could be made of metallic material, preferably stainless steel and/or aluminium. According to an embodiment such grips could have a shape which facilitates the fixing of clusters of nanofibers, for example structures with cylinder arms from 1 to 100, preferably 6, having different and/or equal diameter, preferably between 0.5 and 30 mm of diameter. Such grips could be electrically connected to the ground potential to ease the covering of the ends of the multiscale hierarchical scaffold, during the spinning process, by the sheath of nanofibers according to any embodiments of the herein described process.

In the state of art, in fact, with such homogeneity level there were implemented 1) sheaths electrospun on clusters of twisted nanofibers (yarns) fixed around a drum put in rotation, or 2) sheaths produced apart, inside thereof in a second moment the clusters of axially aligned nanofibers (bundles) and/or clusters of twisted nanofibers (yarns) are inserted or 3) sheaths produced on groups of clusters of axially aligned nanofibers (bundles) and/or clusters of twisted nanofibers (yarns) fixed with each other by a filler such as for example resins. These procedures do not allow high levels of compaction of the final scaffold, which is fundamental to increase the mechanical properties of the construct, and poses serious design constraints on the number of usable clusters of axially aligned nanofibers (bundles) and/or clusters of twisted nanofibers (yarns) and/or ring-like clusters of nanofibers (ring bundles).

The present invention also relates to the ring-like clusters of nanofibers (ring bundles) obtainable according to any one of the embodiments of the herein described process and the production method thereof.

The present invention also relates to a multiscale hierarchical scaffold which can be obtained according to anyone of embodiments of the herein described process.

The herein described multiscale hierarchical scaffold could be used in different applications for example in the biomedical sector in orthopaedic or veterinary field as implantable prosthetic device, in particular if made of biostable material, or for the cellular proliferation and tissue regeneration, in particular if made of bioresorbable material.

If made of biostable synthetic material it could also be applied in robotics and in the production of actuators and guides or in the production of tendons and/or ligaments and/or muscles and/or synthetic nerves for simulating *in vitro* surgical procedures.

A method for the regeneration or replacement of tissues, in particular of the tendinous and/or ligamentous and/or muscular and/or nervous tissue is also herein described, comprising a step of implantation in a subject requiring a multiscale hierarchical scaffold according to anyone of herein described embodiments.

The herein described multiscale hierarchical scaffold could be used in an ex *vivo* method for the production of in *vitro* tendons and/or ligaments and/or muscles and/or nerves, for example a method wherein cells are cultured *in vitro* with the multiscale hierarchical scaffold.

According to an embodiment the present invention also relates to a multiscale scaffold for the replacement, repair, reconstruction or simulation of a tissue, in particular of the tendinous and/or ligamentous and/or muscular and/or nervous tissue comprising:
- a plurality of clusters obtained by electrospinning consisting of axially aligned nanofibers (bundles) and/or clusters of twisted nanofibers (yarns) and/or ring-like clusters of nanofibers (ring bundles) and wherein said plurality of clusters are arranged in order to form one single group;
- a porous sheath obtained by electrospinning consisting of randomly arranged and/or aligned nanofibers, wherein said sheath externally coats and compacts said plurality of clusters keeping them aligned with each other.

The scaffold according to anyone of herein described embodiments advantageously could also be used as sensor implantable in *vivo* or in *vitro,* for example for acquiring and/or transmitting mechanical or physiological signals.

### EXAMPLES

### Example 1

8 prototypes of multiscale hierarchical scaffolds were developed, made of Poly-(L)-lactic acid (PLLA) having length of 100 mm (average diameter 5-6 mm) constituted by 100 clusters of axially aligned nanofibers (bundles) consisting of nanofibers aligned in the direction of the cluster of axially aligned nanofibers (bundle) itself (average diameter of cluster of axially aligned nanofibers (bundles) 550-650 µm, average diameter of nanofibers 500-600 nm).

The sheath was produced thereon by electrospinning the same solution of PLLA used to produce the clusters of axially aligned nanofibers (bundles). The composition is prepared with 13% (weight/volume) of PLLA dissolved in a solvent system of Dichloromethane (DCM) and Dimethylformamide (DMF) in percentage 65/35 (volume/volume). The sheath was produced by electrospinning for 3 hours and by alternating stasis periods of the group of clusters of axially aligned nanofibers (bundles) with rotation periods.

Spinning conditions for the production of one single cluster of axially aligned nanofibers (bundle):
- spinning with 2 metal needles Gauge 20;
- syringe pump delivery 1.2 ml/h;
- electrical field voltage 18 kV;
- rotating collector rotation speed 2900 rpm;
- needle-collector distance 200 mm;
- useful thickness of produced cluster of axially aligned nanofibers (bundle) 550-650 µm

Once the clusters of axially aligned nanofibers (bundles) are cut into samples, each one having length of 100 mm, they were aligned and fixed on the rotating machine for the production of the outer sheath of *random* nanofibers, by applying the following conditions:
- spinning with needle Gauge 20;
- syringe pump delivery 1.2 ml/h;
- electrical field voltage 18 kV;
- metal collector;
- collector-needle(s) distance 200 mm;
- distance between group of clusters of axially aligned nanofibers (bundles) and flat collector smaller than 5 mm,
- rotation speed of the group of clusters of axially aligned nanofibers (bundles) about 20-25 rpm;
- stillness periods of the group of clusters of axially aligned nanofibers (bundles) 2-5 min;
- rotation periods of the group of clusters of axially aligned nanofibers (bundles) 1-2 min;
- useful thickness of the sheath: 5-10 µm

### Example 2: mechanical tests of the produced clusters of axially aligned nanofibers (bundles) obtained in example 1.

Single clusters of axially aligned nanofibers (bundles) were then tested mechanically with a tensile test.

Synthetically the test was performed by using a tensile breaking test with a strain rate of 100% sec⁻¹ for simulating physiological conditions of strain rate compatible to breaking of the tendinous tissue:
- tested samples: 10
- gauge length 16 mm
- crosshead speed 16 mm/sec (strain rate: 1/sec);
- monotonic ramp to break;
- displacement control;
- hydration of the samples before the test for 2 min in 0.9% NaC! saline solution; The single clusters of axially aligned nanofibers (bundles) resisted to breaking up to 4-5 N with a ductile behaviour and deformations in the order of 90%, with an elastic modulus of about 80 MPa.

### Example 3: mechanical tests on produced multiscale hierarchical scaffolds obtained in example 1

The complete multiscale hierarchical scaffolds were tested mechanically, too, with a tensile test even in this case with a strain rate of 100% sec⁻¹ for simulating physiological breaking conditions of the tendinous tissue:
- useful tract 50 mm for 5 tested samples;
- the ends of the samples before the test had been cemented in polymethylmethacrylate (PMMA) for a better clamping. Such casts had tapered shape to minimize the stress concentration.
- crosshead speed 50 mm/sec (strain rate:1/sec);
- monotonic ramp to break;
- displacement control;
- hydration of the samples before the test for 2 min in 0.9% NaC! saline solution; The five multiscale hierarchical scaffolds reached force values between 230 and 380 N, with deformations of about 30% and an elastic modulus of about 130 MPa.

The breaking of samples took place at the interface between a multiscale hierarchical scaffold and cement: this involves that a stress concentration comes up due to the grips which involved a considerable underestimation of the breaking force value of the multiscale hierarchical scaffold.

### Example 4

3 prototypes of multiscale hierarchical scaffolds made of nylon 6,6 with length 230 mm (average diameter 4-5 mm) were developed, constituted by 25 ring-like clusters (ring bundles) consisting of nanofibers aligned in the direction of the axis of the ring-like cluster (ring bundle) (average diameter of bundles 550-650 µm, average diameter of nanofibers 200-300 nm).

The ring-like clusters (ring bundles) were fixed at the ends to the rotating system for the production of sheath, through two grips made of stainless steel constituted by 6 symmetrical cylindrical arms with 8 mm diameter.

On the multiscale hierarchical scaffolds the sheath was produced by electrospinning the same solution of nylon 6,6 used to produce the ring-like clusters (ring bundles). The composition is prepared with 15%(weight/volume) of nylon 6,6 dissolved in a solvent system of Trifluoro-acetic acid (TFA) and Acetone (AC) in percentage 50/50 (volume/volume). The sheath was produced by electrospinning for 12 hours and alternating stasis periods of the group of ring-like bundles (ring bundles) with rotation periods.

Spinning conditions for the production of the single ring-like clusters (ring bundles):
- spinning with 2 metal needles Gauge 20;
- syringe pump delivery 0.5 ml/h;
- electrical field voltage 20 kV;
- rotating collector rotation speed 2900 rpm;
- needle-collector distance 160 mm;
- useful thickness of produced ring-like bundles (ring bundles) 550-650 µm
- length of the ring-like bundles (ring bundles): about 470 mm (deriving from the deposition on a drum with 150 mm diameter)

Once obtained the ring-like clusters (ring bundles), 25 thereof were aligned and fixed at the ends to the arms of the above-described metal grips, on the rotating machine for the production of the outer sheath of random fibers,
by applying the following conditions:
- spinning with 2 needles Gauge 20;
- syringe pump delivery 0.5 ml/h;
- electrical field voltage 18 kV;
- ground flat metal collector;
- collector-needle(s) distance 160 mm;
- distance between group of ring-like clusters of nanofibers (ring bundles) and flat collector smaller than 5 mm;
- rotation speed of the group of ring-like clusters of nanofibers (ring bundles) about 20-25 rpm;
- stillness periods of the group of ring-like clusters of nanofibers (ring bundles) 2-5 min;
- rotation periods of the group of ring-like clusters of nanofibers (ring bundles) 1-2 min;
- useful thickness of the sheath: 5-10 µm
- after 10 hours of sheath spinning on flat collector electrically connected to the ground as previously described, even the two metal grips positioned at the ends of the group of ring-like clusters of nanofibers (ring bundles) were placed to ground potential, so as to coat with the sheath of randomly arranged nanofibers (random) even the ends themselves. The spinning parameters are the same shown above.

### Example 5: mechanical tests of the produced ring-like clusters (ringbundles) obtained in example 4

The single ring-like clusters of nanofibers (ring bundles) were then tested mechanically with a tensile test.

Synthetically the test was performed by using a tensile breaking test with a strain rate of 100% sec⁻¹ to simulate physiological conditions of strain rate compatible to breaking of the tendinous and/or ligamentous and/or muscular and/or nervous tissue:
- tested samples: 5
- gauge length 230 mm
   Crosshead speed 230 mm/sec (strain rate: 1/sec);
- monotonic ramp to break;
- displacement control;
- hydration of the samples before the test for 2 min in 0.9% NaC! saline solution;
The single ring-like clusters of nanofibers (ring bundles) resisted to breaking until 20-24 N with a ductile behaviour and deformations in the order of 9-12%, with an elastic modulus of about 600-900 MPa.

### Example 6: mechanical tests on produced multiscale hierarchical scffolds obtained in example 4

The complete multiscale hierarchical scaffolds, too, were tested mechanically with a tensile test even in this case with a strain rate of 100% sec⁻¹ to simulate breaking physiological conditions of the tendinous and/or ligamentous and/or muscular and/or nervous tissue:
- gauge length 230 mm per 3 tested samples:
- As grips of the samples for the mechanical test, the same metal grips were used therewith the samples were fixed to the machine for the sheath production. Such grips had been planned suitably to deconcentrate the tensions.
- crosshead speed 230 mm/sec (strain rate: 1/sec);
- monotonic ramp to break;
- displacement control;
- hydration of the samples before the test for 2 min in 0.9% NaCl saline solution;

The 3 multiscale hierarchical scaffolds reached force values between 300 and 350 N, with deformations of about 9% and an elastic modulus of about 300 to 400 MPa.

The breaking of the samples took place both at the interface between a multiscale hierarchical scaffold and grips and in the gauge length: this involves that a partial stress concentration comes up due to the grips which involved an underestimation of the breaking force value of the multiscale hierarchical scaffold.

## Claims

1. A multiscale hierarchical scaffold for replacing, repairing, regenerating, reconstructing or simulating a tissue, in particular the tendinous and/or ligamentous and/or muscular and/or nervous tissue comprising:
- a plurality of clusters obtained by electrospinning each one consisting of nanofibers, wherein said plurality of clusters are arranged in order to form one single group;
- a porous sheath obtained by electrospinning consisting of nanofibers, wherein said sheath externally coats and compacts said plurality of clusters keeping them aligned with each other.

2. The scaffold according to claim 1 comprising:
- a plurality of clusters of axially aligned nanofibers (bundles) and/or of clusters of twisted nanofibers (yarns), obtained by electrospinning, consisting of axially aligned and/or twisted nanofibers, respectively, axially arranged so as to form one single group;
- a porous sheath obtained by electrospinning consisting of nanofibers, wherein said sheath externally coats and compacts said plurality of clusters keeping them aligned with each other.

3. The scaffold according to claim 1 or 2 comprising:
- a plurality of ring-like clusters of nanofibers (ring bundles), obtained by electrospinning, consisting of axially aligned and/or axially twisted nanofibers, respectively, and/or arranged randomly so as to form one single group;
- a porous sheath obtained by electrospinning consisting of nanofibers, wherein said sheath externally coats and compacts said plurality of clusters keeping them aligned with each other

4. The scaffold according to any one of claims 1 to 3 having:
- a mechanical resistance comprised between 2 and 10000 N, in particular between 10 and 5000 N and an elastic modulus comprised between 20 and 100000 MPa, in particular between 30 and 20000 MPa; or a mechanical resistance comprised between 200 and 500 N and/or an elastic modulus comprised between 30 and 20000 MPa; or a mechanical resistance comprised between 2 and 10000 N and an elastic modulus comprised between 20 and 100000 MPa .

5. The scaffold according to any one of the preceding claims wherein said scaffold has:
- a length comprised between 10 and 1000 mm, in particular between 10 and 500 mm, and an average diameter comprised between 1 and 100 mm; or a length comprised between 20 and 200 mm and an average diameter comprised between 5 and 50 mm

6. The scaffold according to any one of the preceding claims wherein said nanofibers constituting said cluster and/or said sheath have an average diameter comprised between 200 and 1000 nm, in particular between 300 and 1000 nm.

7. The scaffold according to any one of the preceding claims wherein the average diameter of said clusters is comprised between 1 and 10000 µm, in particular between 20 and 10000 µm.

8. The scaffold comprising a plurality of inner scaffolds according to any one of claims 1 to 7, in turn joined by a second porous sheath obtained by electrospinning consisting of nanofibers, wherein said sheath externally coats and compacts said plurality of scaffolds.

9. The scaffold according to claim 1 to 8 wherein said porous sheath and/or sheaths consists/consist of randomly arranged nanofibers.

10. The scaffold according to claim 1 to 8, wherein said porous sheath and/or sheaths consists/consist of nanofibers arranged aligned axially with respect to the scaffold axis.

11. The scaffold according to claim 1 to 8, wherein said porous sheath and/or sheaths consists/consist of nanofibers arranged with circumferential alignment with respect to the scaffold axis.

12. The scaffold according to any one of claims 8 to 11, wherein said inner scaffolds are axially aligned with one another or wherein said inner scaffolds are twisted with one another (twisting) and/or arranged randomly.

13. The scaffold according to any one of the preceding claims wherein said scaffold is made of polyesters, polyurethanes, polyamides, polyolefins and fluorinated polymers and copolymers thereof or of natural material selected from polysaccharides, proteins, polyesters, polypeptides and copolymers thereof, and/or mixtures thereof, preferably wherein poly-(L)lactic acid (PLLA), polyamides and/or nylon 6,6 is used for preparing the nanofibers.

14. The scaffold according to any one of the preceding claims wherein said scaffold and/or said nanofibers are loaded and/or functionalized with organic and/or inorganic components apt to perform a biological action and/or change in the chemical-physical and/or mechanical properties of said tissue.

15. The scaffold according to any one of the preceding claims wherein the number of said clusters in said scaffold is comprised between 40 and 1000.

16. An implantable prosthetic device comprising a scaffold according to any one of claims 1 to 15, wherein said implantable prosthetic device is a synthetic tendon, a synthetic ligament, a synthetic muscle or a synthetic nerve.

17. A process for preparing a multiscale hierarchical scaffold according to any one of claims 1 to 15 comprising the following steps:
a) preparing by electrospinning a plurality of clusters of axially aligned nanofibers (bundles) and/or clusters of twisted nanofibers (yams) and/or ring-like clusters of nanofibers (ring bundles),
b) electrospinning nanofibers so as to coat said clusters with a porous sheath consisting of nanofibers so as to provide an external coating and to compact the plurality of clusters prepared according to step a).

18. The process for preparing a multiscale hierarchical scaffold according to any one of claims 1 to 15 comprising the following steps:
a) preparing by electrospinning a plurality of clusters of axially aligned nanofibers (bundles) and/or clusters of twisted nanofibers (yarns) and/or ring-like clusters of nanofibers (ring bundles),
b) positioning said plurality of clusters prepared according to step a) so as to form one single group;
c) clamping the group of clusters obtained according to step b) on a grip, capable of axially rotating rigidly and in line, thus, by keeping the clamped group of clusters in a position suitable for the process of coating with electrospun sheath;
d) making by electrospinning a sheath external to the group of clusters clamped at step c), in particular by controlling the rotation parameters of the clamped group of clusters, the geometrical parameters of the setup, and process parameters.

19. The process according to claim 17 or 18 wherein during the step of implementing the sheath the nanofibers are deposited on a collector positioned close to the group of clusters to be coated but having no contact with said group of clusters, in particular it is positioned at about 2-5 mm away from the group of clusters to be coated.

20. The process according to any one of claims 17 to 19 wherein the grips are made of conductive metallic material, for example stainless steel and/or aluminium and positioned at ground potential to improve the deposition of the sheath of nanofibers arranged randomly on the ends of the scaffold itself.

21. The process according to any one of claims 17 to 20, wherein during the step of implementing the sheath the ground collector has a plane geometry or wherein during the step of implementing the sheath the ground collector is a concave, convex or prismatic plate or wherein during the step of implementing the sheath the ground collector consists of two parallel metal rods and/or plates.

22. The process according to any one of claims 17 to 21 comprising the following steps:
a) spinning on rotating drum collector of a plurality of electrospun nanofibers;
b) circumferential winding on the drum of sections of the membrane of electrospun nanofibers to obtain ring-like bundles (ring bundles);
c) removal of the ring-like clusters of nanofibers (ring bundles) from the drum.

23. The process for preparing a multiscale hierarchical scaffold comprising a plurality of inner scaffolds comprising:
a) preparing a plurality of scaffolds according to any one of the preceding claims;
b)electrospinning nanofibers so as to coat said plurality of scaffolds with a porous sheath consisting of nanofibers so as to provide an external coating and to compact the plurality di scaffolds prepared according to step a).

24. Use of a scaffold according to any one of claims 1 to 15 as *in vitro* sensor for acquiring and/or transmitting mechanical or physiological signals

## Patentansprüche

1. Multiskaliges hierarchisches Gerüst zum Ersetzen, Reparieren, Regenerieren, Rekonstruieren oder Simulieren eines Gewebes, insbesondere des Sehnen- und/oder Bänder- und/oder Muskel- und/oder Nervengewebes, umfassend
- eine Vielzahl von durch Elektrospinnen erhaltenen Clustern, die jeweils aus Nanofasern bestehen, wobei die Vielzahl von Clustern so angeordnet ist, dass sie eine einzige Gruppe ausbildet;
- eine poröse Hülle, die durch Elektrospinnen erhalten wird und aus Nanofasern besteht, wobei die Hülle die Vielzahl von Clustern von außen umhüllt und verdichtet und sie zueinander ausgerichtet hält.

2. Gerüst gemäß Anspruch 1 umfasst:
- eine Vielzahl von Clustern axial ausgerichteter Nanofasern (Bündel) und/oder von Clustern verdrehter Nanofasern (Garne), die durch Elektrospinnen erhalten werden und jeweils aus axial ausgerichteten und/oder verdrehten Nanofasern bestehen, die axial so angeordnet sind, dass sie eine einzige Gruppe ausbilden;
- eine poröse Hülle, die durch Elektrospinnen erhalten wird und aus Nanofasern besteht, wobei die Hülle die Vielzahl von Clustern von außen umhüllt und verdichtet und sie zueinander ausgerichtet hält.

3. Gerüst gemäß Anspruch 1 oder 2 umfasst:
- eine Vielzahl von ringartigen Clustern aus Nanofasern (Ringbündel), die durch Elektrospinnen erhalten werden und jeweils aus axial ausgerichteten und/oder axial verdrehten Nanofasern bestehen und/oder zufällig angeordnet sind, so dass sie eine einzige Gruppe ausbilden;
- eine poröse Hülle, die durch Elektrospinnen erhalten wird und aus Nanofasern besteht, wobei die Hülle die Vielzahl von Clustern von außen umhüllt und verdichtet und sie zueinander ausgerichtet hält.

4. Gerüst gemäß einem der Ansprüche 1 bis 3 mit:
- einer mechanischen Festigkeit zwischen 2 und 10000 N, insbesondere zwischen 10 und 5000 N und einem Elastizitätsmodul zwischen 20 und 100000 MPa, insbesondere zwischen 30 und 20000 MPa; oder einer mechanischen Festigkeit zwischen 200 und 500 N und/oder einem Elastizitätsmodul zwischen 30 und 20000 MPa; oder einer mechanischen Festigkeit zwischen 2 und 10000 N und einem Elastizitätsmodul zwischen 20 und 100000 MPa .

5. Gerüst gemäß einem der vorhergehenden Ansprüche, wobei das Gerüst folgendes hat:
- eine Länge zwischen 10 und 1000 mm, insbesondere zwischen 10 und 500 mm, und einen mittleren Durchmesser zwischen 1 und 100 mm liegend; oder eine Länge zwischen 20 und 200 mm und einen mittleren Durchmesser zwischen 5 und 50 mm liegend.

6. Gerüst gemäß einem der vorhergehenden Ansprüche, wobei die Nanofasern, die den Cluster und/oder die Hülle bilden, einen mittleren Durchmesser haben, der zwischen 200 und 1000 nm, insbesondere zwischen 300 und 1000 nm, liegt.

7. Gerüst gemäß einem der vorhergehenden Ansprüche, wobei der mittlere Durchmesser der Cluster zwischen 1 und 10000 µm, insbesondere zwischen 20 und 10000 µm, liegt.

8. Gerüst, umfassend eine Vielzahl von inneren Gerüsten gemäß einem der Ansprüche 1 bis 7, die ihrerseits durch eine zweite poröse Hülle verbunden sind, welche durch Elektrospinnen erhalten wurde und aus Nanofasern besteht, wobei die Hülle die Vielzahl von Gerüsten von außen umhüllt und verdichtet.

9. Gerüst gemäß den Ansprüchen 1 bis 8, wobei die poröse Hülle und/oder die porösen Hüllen aus zufällig angeordneten Nanofasern besteht/bestehen.

10. Gerüst gemäß einem der Ansprüche 1 bis 8, wobei die poröse Hülle und/oder die porösen Hüllen aus Nanofasern besteht/bestehen, die in Bezug auf die Gerüstachse axial ausgerichtet sind.

11. Gerüst gemäß Anspruch 1 bis 8, wobei die poröse Hülle und/oder die porösen Hüllen aus Nanofasern besteht/bestehen, die in Umfangsrichtung in Bezug auf die Gerüstachse ausgerichtet sind.

12. Gerüst gemäß einem der Ansprüche 8 bis 11, wobei die inneren Gerüste axial zueinander ausgerichtet sind oder wobei die inneren Gerüste miteinander verdreht (Verdrehung) sind und/oder zufällig angeordnet sind.

13. Gerüst gemäß einem der vorhergehenden Ansprüche, wobei das Gerüst aus Polyestern, Polyurethanen, Polyamiden, Polyolefinen und fluorierten Polymeren und Copolymeren davon oder aus natürlichem Material, ausgewählt aus Polysacchariden, Proteinen, Polyestern, Polypeptiden und Copolymeren davon und/oder Mischungen davon, hergestellt ist, wobei vorzugsweise Poly-(L)milchsäure (PLLA), Polyamide und/oder Nylon 6,6 zur Herstellung der Nanofasern verwendet wird.

14. Gerüst gemäß einem der vorhergehenden Ansprüche, wobei das Gerüst und/oder die Nanofasern mit organischen und/oder anorganischen Komponenten beladen und/oder funktionalisiert sind, die eine biologische Wirkung und/oder eine Veränderung der chemisch-physikalischen und/oder mechanischen Eigenschaften des Gewebes auslösen können.

15. Gerüst gemäß einem der vorhergehenden Ansprüche, wobei die Anzahl der Cluster in dem Gerüst zwischen 40 und 1000 umfasst ist.

16. Implantierbare prothetische Vorrichtung, umfassend ein Gerüst gemäß einem der Ansprüche 1 bis 15, wobei die implantierbare prothetische Vorrichtung eine synthetische Sehne, ein synthetisches Band, ein synthetischer Muskel oder ein synthetischer Nerv ist.

17. Verfahren zur Herstellung eines multiskaligen hierarchischen Gerüsts gemäß einem der Ansprüche 1 bis 15, umfassend die folgenden Schritte:
a) Herstellen einer Vielzahl von Clustern aus axial ausgerichteten Nanofasern (Bündeln) und/oder Clustern aus verdrehten Nanofasern (Garnen) und/oder ringartigen Clustern aus Nanofasern (Ringbündeln) durch Elektrospinnen;
b) Elektrospinnen von Nanofasern, um die Cluster mit einer porösen Hülle zu beschichten, die aus Nanofasern besteht, um eine äußere Beschichtung bereitzustellen und die gemäß Schritt a) hergestellte Vielzahl von Clustern zu verdichten.

18. Verfahren zur Herstellung eines multiskaligen hierarchischen Gerüsts gemäß einem der Ansprüche 1 bis 15, umfassend die folgenden Schritte:
a) Herstellen einer Vielzahl von Clustern aus axial ausgerichteten Nanofasern (Bündeln) und/oder Clustern aus verdrehten Nanofasern (Garnen) und/oder ringartigen Clustern aus Nanofasern (Ringbündeln) durch Elektrospinnen;
b) Positionieren der gemäß Schritt a) hergestellten Vielzahl von Clustern, um eine einzige Gruppe auszubilden;
c) Einspannen der gemäß Schritt b) erhaltenen Gruppe von Clustern auf einer Klemme, die in der Lage ist, sich axial starr und in einer Linie zu drehen, wodurch die eingespannte Gruppe von Clustern in einer Position gehalten wird, die für den Prozess des Beschichtens mit einem elektrogesponnenen Hülle geeignet ist;
d) Herstellen einer äußeren Hülle der in Schritt c) eingespannten Gruppe von Clustern durch Elektrospinnen, insbesondere durch Steuern der Rotationsparameter der eingespannten Gruppe von Clustern, der geometrischen Parameter des Aufbaus und der Prozessparameter.

19. Verfahren gemäß Anspruch 17 oder 18, wobei die Nanofasern während des Schritts der Realisierung der Hülle auf einem Kollektor abgeschieden werden, der in der Nähe der zu beschichtenden Clustergruppe positioniert ist, aber keinen Kontakt mit der Clustergruppe hat, der insbesondere in einem Abstand von etwa 2-5 mm von der zu beschichtenden Clustergruppe entfernt positioniert ist.

20. Verfahren gemäß einem der Ansprüche 17 bis 19, wobei die Klemmen aus leitfähigem metallischem Material, z. B. rostfreiem Stahl und/oder Aluminium, hergestellt und auf einem Erdpotential liegen, um das Abscheiden der Hülle aus Nanofasern zu verbessern, die zufällig an den Enden des Gerüsts selbst angeordnet sind.

21. Verfahren gemäß einem der Ansprüche 17 bis 20,
wobei während des Schritts des Realisierens der Hülle der Erdkollektor eine ebene Geometrie hat oder wobei während des Schritts des Realisierens der Hülle der Erdkollektor eine konkave, konvexe oder prismatische Platte ist oder wobei während des Schritts des Realisierens der Hülle der Erdkollektor aus zwei parallelen Metallstäben und/oder -platten besteht.

22. Verfahren gemäß einem der Ansprüche 17 bis 21, umfassend die folgenden Schritte:
a) Spinnen einer Vielzahl von elektrogesponnenen Nanofasern auf einem rotierenden Trommelkollektor;
b) Aufwickeln von Abschnitten der Membran aus elektrogesponnenen Nanofasern in Umfangsrichtung auf der Trommel, um ringartige Bündel (Ringbündel) zu erhalten;
c) Entfernen der ringartigen Bündel von Nanofasern (Ringbündel) von der Trommel. 1

23. Verfahren zur Herstellung eines multiskaligen hierarchischen Gerüsts, das eine Vielzahl von inneren Gerüsten umfasst, umfassend:
a) Herstellen einer Vielzahl von Gerüsten gemäß einem der vorhergehenden Ansprüche;
b) Elektrospinnen von Nanofasern, um die Vielzahl von Gerüsten mit einer porösen Hülle, die aus Nanofasern besteht, zu beschichten, um eine äußere Beschichtung bereitzustellen und die gemäß Schritt a) hergestellte Vielzahl von Gerüsten zu verdichten.

24. Verwendung eines Gerüsts gemäß einem der Ansprüche 1 bis 15 als In-vitro-Sensor zum Erfassen und/oder Übertragen mechanischer oder physiologischer Signale

## Revendications

1. Échafaudage hiérarchique à structure multi-échelles pour le remplacement, la réparation, la régénération, la reconstruction ou la simulation d'un tissu, en particulier du tissu tendineux et/ou ligamenteux et/ou musculaire et/ou nerveux, comprenant :
- une pluralité de clusters obtenus par électrofilage de chacun se composant de nanofibres, ladite pluralité de clusters étant disposée afin de former un groupe unique ;
- une gaine poreuse obtenue par électrofilage se composant de nanofibres, ladite gaine revêtant en externe et compactant ladite pluralité de clusters en les gardant alignés les uns par rapport aux autres.

2. Échafaudage selon la revendication 1, comprenant :
- une pluralité de clusters de nanofibres alignées axialement (faisceaux) et/ou clusters de nanofibres torsadées (fils), obtenus par électrofilage, se composant de nanofibres alignées axialement et/ou torsadées, respectivement, disposés axialement de manière à former un groupe unique ;
- une gaine poreuse obtenue par électrofilage se composant de nanofibres, ladite gaine revêtant en externe et compactant ladite pluralité de clusters en les gardant alignés les uns par rapport aux autres.

3. Échafaudage selon la revendication 1 ou 2, comprenant :
- une pluralité de clusters de type annulaire de nanofibres (faisceaux annulaires), obtenus par électrofilage, se composant de nanofibres alignées axialement et/ou torsadées, respectivement, et/ou disposées aléatoirement de manière à former un groupe unique ;
- une gaine poreuse obtenue par électrofilage se composant de nanofibres, ladite gaine revêtant en externe et compactant ladite pluralité de clusters en les gardant alignés les uns par rapport aux autres.

4. Échafaudage selon l'une quelconque des revendications 1 à 3, ayant :
- une résistance mécanique comprise entre 2 et 10000 N, en particulier entre 10 et 5000 N, et un module élastique compris entre 20 et 100000 MPa, en particulier entre 30 et 20000 MPa ; ou une résistance mécanique comprise entre 200 et 500 N et/ou un module élastique compris entre 30 et 20000 MPa ; ou une résistance mécanique comprise entre 2 et 10000 N et un module élastique compris entre 20 et 100000 MPa.

5. Échafaudage selon l'une quelconque des revendications précédentes, ledit échafaudage ayant :
- une longueur comprise entre 10 et 1000 mm, en particulier entre 10 et 500 mm, et un diamètre moyen compris entre 1 et 100 mm ; ou une longueur comprise entre 20 et 200 mm et un diamètre moyen compris entre 5 et 50 mm.

6. Échafaudage selon l'une quelconque des revendications précédentes, dans lequel lesdites nanofibres composant ledit cluster et/ou ladite gaine ont un diamètre moyen compris entre 200 et 1000 nm, en particulier entre 300 et 1000 nm.

7. Échafaudage selon l'une quelconque des revendications précédentes, dans lequel le diamètre moyen desdits clusters est compris entre 1 et 10000 µm, en particulier entre 20 et 10000 pm.

8. Échafaudage comprenant une pluralité d'échafaudages internes selon l'une quelconque des revendications 1 à 7, rassemblés à leur tour par une seconde gaine poreuse obtenue par électrofilage se composant de nanofibres, dans lequel ladite gaine revêt en externe et compacte ladite pluralité d'échafaudages.

9. Échafaudage selon les revendications 1 à 8, dans lequel ladite gaine poreuse et/ou lesdites gaines poreuses se compose/composent de nanofibres disposées aléatoirement.

10. Échafaudage selon les revendications 1 à 8, dans lequel ladite gaine poreuse et/ou lesdites gaines poreuses se compose/composent de nanofibres disposées alignées axialement par rapport à l'axe de l'échafaudage.

11. Échafaudage selon les revendications 1 à 8, dans lequel ladite gaine poreuse et/ou lesdites gaines poreuses se compose/composent de nanofibres disposées avec un alignement circonférentiel par rapport à l'axe de l'échafaudage.

12. Échafaudage selon l'une quelconque des revendications 8 à 11, dans lequel lesdits échafaudages internes sont alignés axialement les uns par rapport aux autres ou dans lequel lesdits échafaudages sont torsadés les uns avec les autres (torsade) et/ou disposés aléatoirement.

13. Échafaudage selon l'une quelconque des revendications précédentes, ledit échafaudage étant fait de polyesters, de polyuréthanes, de polyamides, de polyoléfines, et de polymères et copolymères fluorés de ceux-ci ou de matière naturelle choisie parmi les polysaccharides, les protéines, les polyesters, les polypeptides, et des copolymères de ceux-ci, et/ou des mélanges de ceux-ci, l'acide poly-(L)-lactique (PLLA), les polyamides et/ou le nylon 6-6 étant de préférence utilisés pour préparer les nanofibres.

14. Échafaudage selon l'une quelconque des revendications précédentes, ledit échafaudage et/ou lesdites nanofibres étant chargés et/ou fonctionnalisés avec des composants organiques et/ou inorganiques capables de réaliser une action biologique et/ou un changement biologique dans les propriétés physico-chimiques et/ou mécaniques dudit tissu.

15. Échafaudage selon l'une quelconque des revendications précédentes, dans lequel le nombre desdits clusters dans ledit échafaudage est compris entre 40 et 1000.

16. Dispositif prothétique implantable comprenant un échafaudage selon l'une quelconque des revendications 1 à 15, ledit dispositif implantable étant un tendon synthétique, un ligament synthétique, un muscle synthétique ou un nerf synthétique.

17. Procédé de préparation d'un échafaudage hiérarchique à structure multi-échelles selon l'une quelconque des revendications 1 à 15, comprenant les étapes suivantes :
a) la préparation par électrofilage d'une pluralité de clusters de nanofibres alignées axialement (faisceaux) et/ou clusters de nanofibres torsadées (fils) et/ou clusters de type annulaire de nanofibres (faisceaux annulaires) ;
b) l'électrofilage des nanofibres de manière à revêtir lesdits clusters avec une gaine poreuse se composant de nanofibres de manière à fournir un revêtement externe et à compacter la pluralité de clusters préparés selon l'étape a).

18. Procédé de préparation d'un échafaudage hiérarchique à structure multi-échelles selon l'une quelconque des revendications 1 à 15, comprenant les étapes suivantes :
a) la préparation par électrofilage d'une pluralité de clusters de nanofibres alignées axialement (faisceaux) et/ou clusters de nanofibres torsadées (fils) et/ou clusters de type annulaire de nanofibres (faisceaux annulaires) ;
b) le positionnement de ladite pluralité de clusters préparés selon l'étape a) de manière à former un groupe unique ;
c) le serrage du groupe de clusters obtenus selon l'étape b) sur un dispositif de prise, capable de tourner axialement de manière rigide et en ligne, maintenant ainsi le groupe serré de clusters dans une position appropriée pour le processus de revêtement avec la gaine électrofilée ;
d) la fabrication par électrofilage d'une gaine externe sur le groupe de clusters serrés à l'étape c), en particulier en contrôlant les paramètres de rotation du groupe serré de clusters, les paramètres géométriques du réglage et les paramètres de processus.

19. Procédé selon la revendication 17 ou 18, dans lequel, au cours de l'étape de mise en place de la gaine, les nanofibres sont déposées sur un collecteur positionné près du groupe de clusters à revêtir mais sans avoir de contact avec ledit groupe de clusters, en particulier, positionné à environ 2 à 5 mm de distance du groupe de clusters à revêtir.

20. Procédé selon l'une quelconque des revendications 17 à 19, dans lequel les dispositifs de prise sont fabriqués en matériau métallique conducteur, par exemple en acier inoxydable et/ou aluminium et sont positionnés au niveau du potentiel de terre pour améliorer le dépôt de la gaine de nanofibres disposées aléatoirement sur les extrémités dudit échafaudage lui-même.

21. Procédé selon l'une quelconque des revendications 17 à 20, dans lequel, au cours de l'étape de mise en place de la gaine, le collecteur mis à la terre a une géométrie plane ou dans lequel, au cours de l'étape de mise en place de la gaine, le collecteur mis à la terre est une plaque concave, convexe ou prismatique ou dans lequel, au cours de l'étape de mise en place de la gaine, le collecteur mis à la terre se compose de deux tiges et/ou plaques métalliques parallèles.

22. Procédé selon l'une quelconque des revendications 17 à 21, comprenant les étapes suivantes :
a) le filage sur un collecteur à tambour rotatif d'une pluralité de nanofibres électrofilées ;
b) l'enroulement circonférentiel sur le tambour de sections de la membrane de nanofibres électrofilées pour obtenir des faisceaux de type annulaire (faisceaux annulaires) ;
c) le retrait des clusters de type annulaire de nanofibres (faisceaux annulaires) du tambour.

23. Procédé de préparation d'un échafaudage hiérarchique à structure multi-échelles comprenant une pluralité d'échafaudages internes, comprenant :
a) la préparation d'une pluralité d'échafaudages selon l'une quelconque des revendications précédentes ;
b) l'électrofilage de nanofibes de manière à revêtir ladite pluralité d'échafaudages avec une gaine poreuse se composant de nanofibres de manière à fournir un revêtement externe et à compacter la pluralité d'échafaudages préparés selon l'étape a).

24. Utilisation d'un échafaudage selon l'une quelconque des revendications 1 à 15 en tant que capteur *in vitro* pour l'acquisition et/ou la transmission de signaux mécaniques ou physiologiques.
